# EUROPEAN PATENT APPLICATION

(11) **EP 3 342 351 A1**
(43) Date of publication of application: **04.07.2018**
(21) Application number: 17207321.5
(22) Date of filing: 14.12.2017
(51) Int. Cl.: A61B 6/06, A61B 6/00, G21K 1/04, G21K 1/02

(54) **COMPACT INTERCHANGEABLE FILTERS MECHANISM**

(30) Priority: 15.12.2016 US 201662434443 P; 29.12.2016 US 201662439924 P
(71) Applicant: ControlRAD Systems Inc., Radnor, Pennsylvania 19087 (US)
(72) Inventor: Melman, Haim Zvi, 4433512 Israel (IL)
(74) Representative: Harrison, Robert John

(57) **Abstract**

In a multiple frame x-ray imaging system, multiple stacked interchangeable filters mounted in a housing, each filter comprising a Region of Interest (ROI) aperture, said multiple filters comprising at least two filters having different Region of Interest (ROI) aperture shapes.

## Description

The present invention is in the field of radiation filters and more particularly configurations of interchangeable filters.

This patent application claims priority from and is related to U.S. Provisional Patent Application Serial Number 62/434,443, filed 12/15/2016 and U.S. Provisional Patent Application Serial Number 62/439,924, filed 12/29/2016, these U.S. Provisional Patent Applications are incorporated by reference in their entirety herein.

In a typical multiple frame x-ray imaging system, the x-ray tube generates x-ray radiation over a relatively wide solid angle. To avoid unnecessary exposure to both the patient and the medical team, collimators of x-ray absorbing materials such as lead are used to block the redundant radiation. This way only the necessary solid angle of useful radiation exits the x-ray tube assembly to expose only the necessary elements.

In multiple frame x-ray imaging (such as fluoroscopy) the situation is more dynamic than in a single exposure x-ray. The x-ray radiation is active for relatively long period. The patient is exposed to prolonged direct x-ray radiation and the treating physician typically has to stand near the patient, therefore near the x-ray radiation where he is exposed to prolonged scattered radiation. As a result, it is desired to provide methods to minimize exposure to the patient and medical team. Methods for reducing x-ray radiation intensity have been suggested where the resultant reduced signal to noise ratio (S/N) of the x-ray image is compensated by digital image enhancement. Other methods suggest a filter limiting the solid angle of the full x-ray radiation to a fraction of the image intensifier area and moving the filter to swap the input area of the image intensifier where the Region of Interest (ROI) is exposed more than the rest of the area. This way, the ROI gets high enough x-ray radiation to generate a good S/N image while the rest of the image is exposed with low x-ray intensity, providing a relatively low S/N image. The ROI size and position can be determined in a plurality of methods.

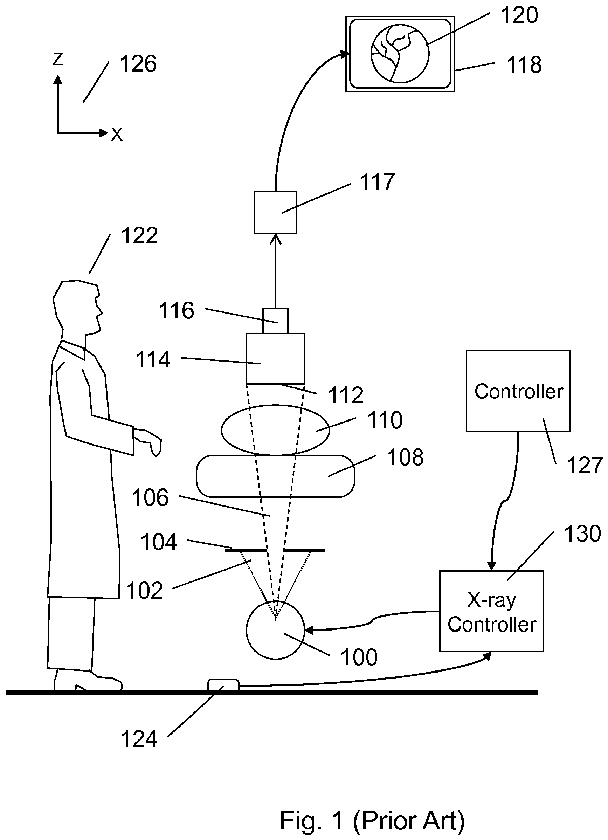

Fig. 1 presents a typical layout of a fluoroscopy clinical environment. X-ray tube 100 generates x-ray radiation 102 directed upward occupying a relatively large solid angle towards collimator 104. Collimator 104 blocks a part of the radiation allowing a smaller solid angle of radiation 106 to continue in the upward direction, go through bed 108 that is typically made of material that is relatively transparent to x-ray radiation and through patient 110 who is laying on bed 108. Part of the radiation is absorbed and scattered by the patient and the remaining radiation arrives at the typically round input area 112 of image intensifier 114. The image generated by image intensifier 114 is captured by camera, 116 processed by image processor 117 and then displayed on monitor 118 as image 120.

Operator 122 is standing by the patient to perform the medical procedure while watching image 120.

The operator has a foot-switch 124. When pressing the switch, continuous x-ray radiation (or pulsed x-ray as explained below) is emitted to provide a cine imaging 120. The intensity of x-ray radiation is typically optimized in a tradeoff of low intensity that is desired to reduce exposure to the patient and the operator and high intensity radiation that is desired to enable a high quality image 120 (high S/N). With low intensity x-ray radiation and thus low exposure of the image intensifier input area, the S/N of image 120 might be so low that image 120 becomes useless.

Coordinate system 126 is a reference Cartesian coordinate system with Y axis pointing into the page and X-Y is a plane parallel to planes such as that of collimator 104 and image intensifier input plane 112.

Various means may be used to selectively radiate different areas of the scene with different radiation intensity or Dose Per Pixel (DPP). These means may comprise an Eye tracker or any alternative input device provides indication where operator 122 is looking. This information is typically provided relative to monitor 118.

Image processing is used to at least brighten up the image outside of the ROI that is dark due to low radiation resulting from the filtering of the x-ray in this image part, thereby the background image becomes more similar (in at least brightness) to the image inside the ROI. The image processing may be done in various methods such as, tone correction.

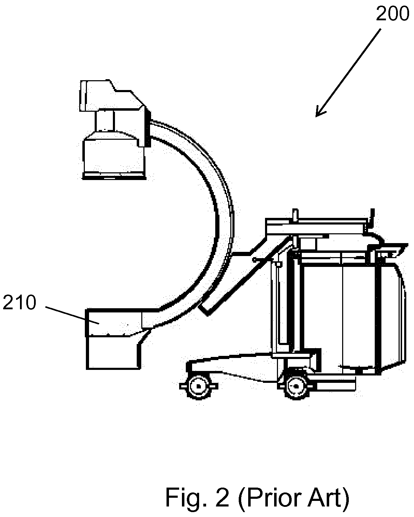

Fig. 2 is a schematic representation of a mobile C-arm fluoroscopy X-ray system 200 used for a variety of diagnostic imaging and minimally invasive surgical procedures. Some systems offer technologies to reduce the radiation dose delivered to patients, by introducing dose reduction filters into the space 210 formed in the C-arm base. These systems do not comprise multiple interchangeable dose reduction filters with multiple ROIs of different shapes with a simple replacement support of one set of filters with another set of filters.

The present invention provides, in a multiple frame x-ray imaging system, multiple stacked interchangeable filters mounted in a housing, each filter comprising a Region of Interest (ROI) aperture, said multiple filters comprising at least two filters having different Region of Interest (ROI) aperture shapes.

At least one of said ROI aperture's shape is application specific.

The application may comprise spine radiation and the ROI aperture may be an elongated slit.

The application may comprise coronary arteries radiation and the ROI aperture may be moon-shaped.

The application may comprise kidney radiation and the ROI aperture may be elliptical.

The multiple filters may include at least two filters providing different radiation dose reductions.

The at least two filters providing different radiation dose reductions may have different thicknesses.

The at least two filters providing different radiation dose reductions may be made of different materials.

At least one of the multiple filters may comprise a ROI with sharp edges.

At least one of the multiple filters may comprise a ROI with edges comprising gradually changing thickness.

At least one of the multiple filters may comprise a ROI with irregular shape.

The system may further comprise a drawing mechanism configured to draw selected one or more filters in a first horizontal direction inside said housing for selectively reducing radiation.

The drawing mechanism may comprise independent slider crank mechanisms for each filter, the crank mechanisms driven by respective motors.

The drawing mechanism may comprise independent rack and pinion gear sets for each filter, the rack and pinion gear sets, driven by respective motors.

The drawing mechanism may comprise independent leadscrews for each filter, the leadscrews actuated by leadscrew actuators.

The selected one or more filters may comprise more than one filter, whereby an active ROI aperture shape is created by the intersection of the ROI shapes of the more than one filter.

The system may further comprise a mechanism for moving the housing in a second horizontal direction, essentially perpendicular to the first horizontal direction.

At least one of the plurality of filters may be replaceable.

The drawing mechanism may be configured to slide the filters so that the center of at least one ROI does not coincide with the center of the radiated beam.

For better understanding of the invention and to show how the same may be carried into effect, reference will now be made, purely by way of example, to the accompanying drawings.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
Fig. 1 presents a typical layout of a fluoroscopy clinical environment;
Fig. 2 is a schematic representation of a mobile C-arm fluoroscopy X-ray system;
Figs. 3A and 3B are schematic illustrations of a first embodiment of the interchangeable filters;
Figs. 4A and 4B are schematic illustrations of a second embodiment of the interchangeable filters;
Figs. 5A and 5B are schematic illustrations of a third embodiment of the interchangeable filters;
Fig. 6 is an example of a variety of filters that can be used in the embodiments of the invention; and
Fig. 7 is an illustration of an embodiment example of hybrid filters of the present invention.

The present invention solves the problem of introducing multiple interchangeable dose reduction filters, each including a different Region of Interest (ROI) aperture for focusing x-ray radiation onto a desired location and reducing peripheral radiation.

Each filter may also provide a different dose reduction level based on thickness or material or both.

It also provides for a simple change of each filter element with another filter element to support a request to change the shape of the ROI aperture, the dose reduction level or both.

In each of the following examples three filters are shown. It will be appreciated that the same concept may be used to interchange two or more filters, depending on the application.

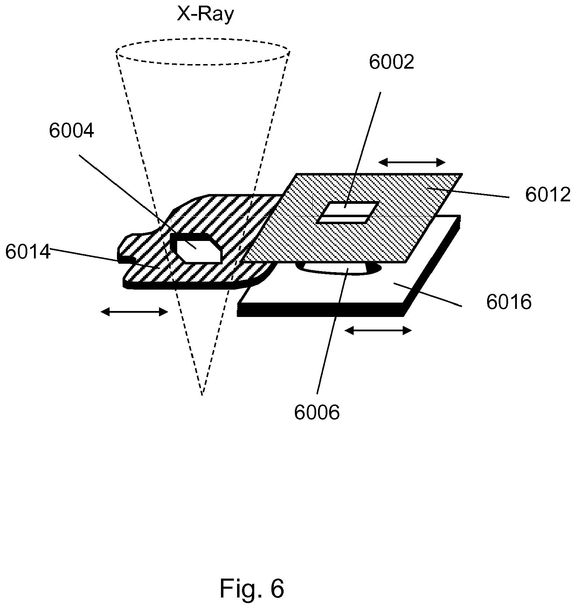

In the examples of Fig. 2A through 5B both filters and ROI apertures are shown as rectangular. It will be appreciated, as shown in Fig. 6, that the same concept may be used with any shape of filter, shape of ROI aperture, filter material and filter thickness.

A particular embodiment of the invention is enabling shapes that suit specific applications, for example a long narrow rectangular aperture for spine surgery and a relatively small-oval ROI aperture for aneurism coil deployment.

In the following examples the filter plates are assumed to be partially transparent to radiation. It will be appreciated that the same concept may be used with opaque filter plates.

In the following examples the different mechanisms are shown to draw one filter plate at a time, namely only one plate is operable to selectively filter radiation at any given moment. It will be appreciated that two or more plates may be operable simultaneously, thus creating, in combination, various filtering levels and ROI apertures defined by the intersection of the ROI apertures of the simultaneously operable filters and various shaped filters with varying degrees of dose reduction defined by the additive dose reduction of overlapping areas of the simultaneously operable filters.

Such filters may be constructed from various materials such as copper, aluminum and titanium, with thickness depending on the amount of dose reduction desired. For example, 0.8mm copper will reduce radiation in about 90% at 55KVp (depending on the pre-filtering of the x-ray system).

The filter drawing mechanisms are configured to draw the filters so that the center ROI does not necessarily coincide with the center of the radiated beam.

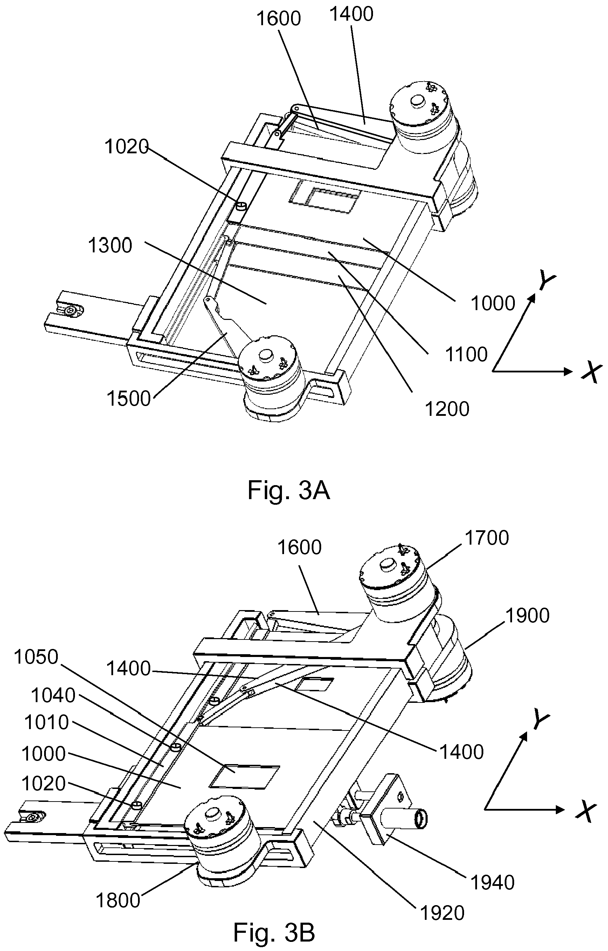

Figs. 3A and 3B are schematic illustrations of a first embodiment of the interchangeable filters.

In Fig. 3A the three filter plates 1000, 1100 and 1200 are shown stacked in a retracted position, namely, no filter is in use and the radiation is free to flow through opening 1300.

Screws 1020, 1040 (Fig. 3B) hold the filter 1000 onto filter holder 1010. Similar screws hold the other filters to respective filter holders. By removing the screws another filter can be installed with a different filtration degree (material and/or thickness), different shape and/or a different ROI aperture

Three independent slider crank mechanisms 1400, 1500 and 1600 manipulate respectively the three plates in direction parallel to Y. With this arrangement the ROI aperture can be moved in the Field of View (FOV), at opening 1300, to be positioned in a location desired by the user in the FOV. The crank mechanisms are driven using three respective motors 1700, 1800 and 1900 (such as, for example, Faulhaber DC Gearmotor 2619).

In Fig. 3B filter plate 1000 is shown covering the opening 1300, so that unfiltered radiation may only flow through ROI aperture 1050 while the rest of the radiation is reduced when passing through the filter material.

In the embodiment of Fig. 3B motor and axis 1940 are added. These enable the movement of the assembly frame 1920 in direction parallel to X, thus controlling the location of the filters in the FOV also in direction parallel to X.

With this arrangement the user can place the ROI aperture anywhere in the FOV. It is a matter of designing the movement range in X and Y so to enable any range for ROI position in the FOV.

By releasing screws 1020 and 1040 filter 1000 can be removed and be replaced by another filter, as desired by the user. Same embodiment of filter replacement is provided for filters 1100 and 1200.

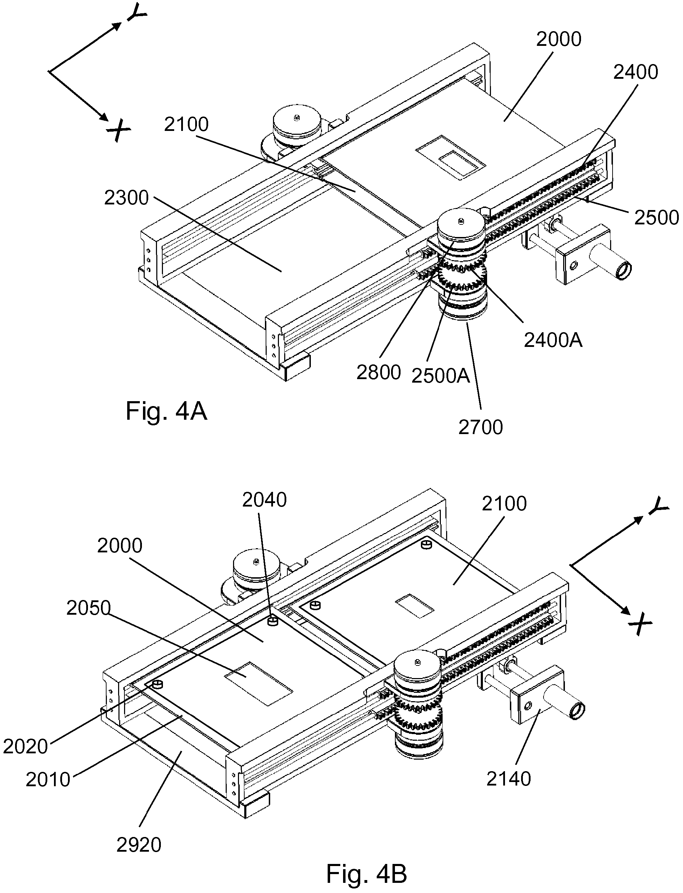

Figs. 4A and 4B are schematic illustrations of a second embodiment of the interchangeable filters.

In Fig. 4A an example of two filters is shown. The two filter plates 2000 and 2100 are stacked in a retracted position, namely, no filter is in use and the radiation is free to flow through opening 2300.

Screws 2020, 2040 (Fig. 4B) hold the filter 2000 onto a frame 2010. Similar screws hold the other filter to its frame. By removing the screws another filter can be installed with a different filtration degree, different shape and/or a different ROI aperture

Two independent rack and pinion gear sets (2400; 2400A) and (2500; 2500A) manipulate respectively the two plates in direction parallel to Y. With this arrangement the ROI aperture can be moved in the Field of View (FOV) to be positioned in a location desired by the user in the FOV. The rack and pinion gear sets are driven using two respective motors 2700 and 2800 (such as, for example, Maxon 241916).

In Fig. 4B filter plate 2000 is shown covering the opening 2300, so that unfiltered radiation may only flow through ROI aperture 2050 while the rest of the radiation is reduced when passing through the filter material.

In the embodiment of Fig. 4B motor and axis 2140 are added. These enable the movement of the entire frame 2920 in direction parallel to X, thus controlling the location of the filters in the FOV also in direction parallel to X.

With this arrangement the user can place the ROI aperture anywhere in the FOV.

By releasing screws 2020 and 2040 filter 2000 can be removed and be replaced by another filter, as desired by the user. Same embodiment of filter replacement is provided for filter 2100.

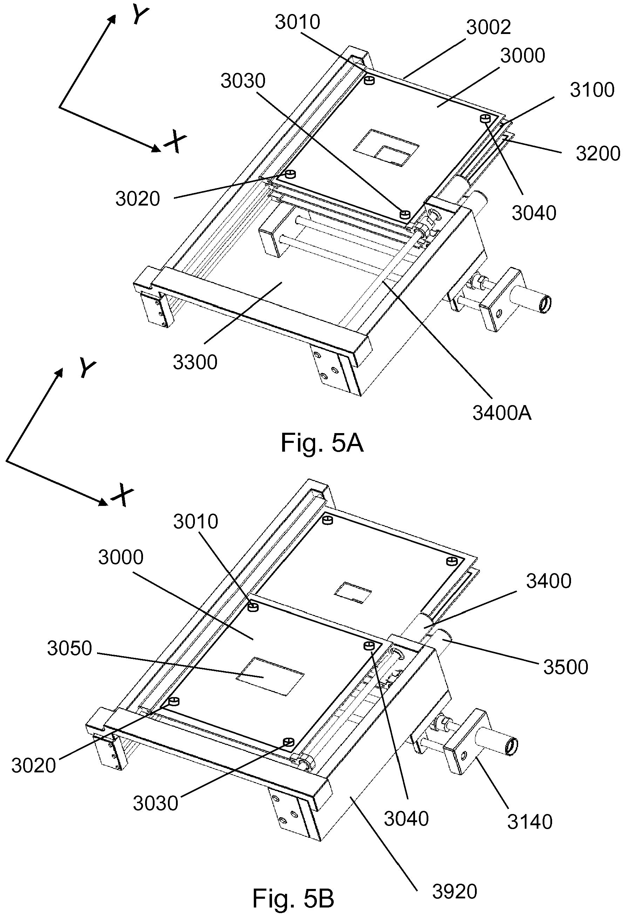

Figs. 5A and 5B are schematic illustrations of a third embodiment of the interchangeable filters.

In Fig. 5A the three filter plates 3000, 3100 and 3200 are shown stacked in a retracted position, namely, no filter is in use and the radiation is free to flow through opening 3300.

Screws 3010, 3020, 3030 and 3040 hold the filter 3000 onto frame 3002. Similar screws hold the other filters to respective frames. By removing the screws another filter can be installed with a different filtration degree and/or a different ROI aperture

Three independent leadscrew actuators 3400, 3500 and 3600 (such as, for example, Maxon Spindle Drive GP 8 S) (only two seen) control respectively three leadscrews 340A, 350A and 360A (only one seen) to manipulate respectively the three plates in direction parallel to Y. With this arrangement the ROI aperture can be moved in the Field of View (FOV) to be positioned in a location desired by the user in the FOV.

In Fig. 5B filter plate 3000 is shown positioned in the opening 3300, so that unfiltered radiation may only flow through ROI aperture 3050 while the rest of the radiation is reduced when passing through the filter material.

In the embodiment of Fig. 5B motor and axis 3140 are added. These enable the movement of the entire assembly frame 3920 in direction parallel to X, thus controlling the location of the filters in the FOV also in direction parallel to X.

With this arrangement the user can place the ROI aperture anywhere in the FOV.

By releasing screws 3010, 3020, 3030 and 3040 filter 3000 can be removed and replaced by another filter, as desired by the user. Same embodiment of filter replacement is provided for filters 3100 and 3200.

Fig. 6 is an example of a variety of filters that can be used in the embodiments of the invention.

In this example, filter 6012 is made from a first material, it is the thinnest filter among the three filters of Fig. 6, the shape of the filter is rectangular and it has a rectangular ROI 6002.

filter 6014 is made from a second material, it is thicker than filter 6012 but thinner than filter 6016, the shape of the filter is irregular smooth contour and it has an irregular ROI shape 6004 of straight edges.

Filter 6016 is made from a third material, it is the thickest filter among the three filters of Fig. 6, the shape of the filter is rectangular and it has an oval ROI 6006.

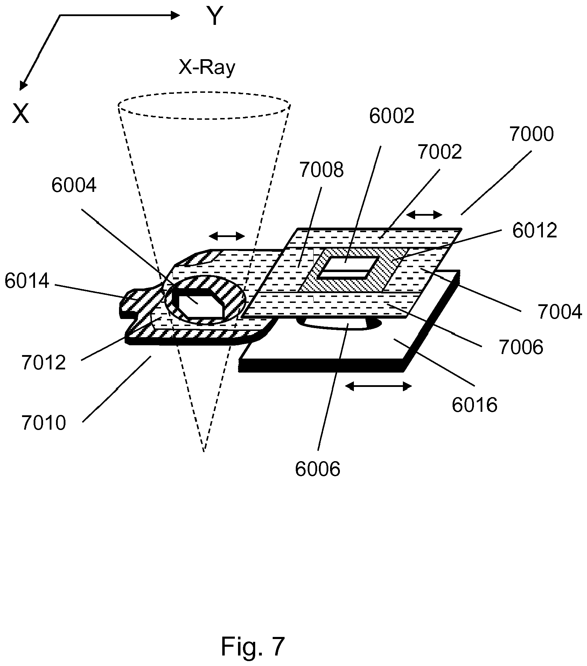

In yet another embodiment example, it might be desired to assemble the filters of the present invention inside an existing collimator where the design of the collimator does not provide enough space to insert such filters. In the present embodiment, a part of the existing collimator is removed. For example, the collimation blades (and collimator blades actuator mechanism, if so needed) can be removed from the collimator and the filters of the present invention can be inserted in the vacancy resulting from the removal of the collimator blades (and related mechanism/parts). To overcome possible need to limit the x-ray beam to a specific area of the detector, an x-ray limiting material (typically lead or tungsten) can be attached to the filters of the present invention so that it becomes a hybrid of at least two materials, to provide the beam limiting function that was handled by the collimator blades before they were removed. Fig. 7 illustrates such a hybrid filter 7000. It comprises dose reduction filter 6012 that is partially transparent to x-ray with ROI 6002. In the example of Fig. 7, additional layer of beam limiting material 7002, 7004, 7006 and 7008 was added. Therefore, this layer provides the function of the collimator blades as required, section 6012 provide the low radiation for the background image and ROI 6002 provides the full radiation higher quality image part.

In the example of hybrid filter 7000 the area that is not blocked by beam limiting material 7002, 7004, 7006 and 7008 is rectangle as is common in flat panel detectors based c-arms. In the example of hybrid filter 7010 the opening in the beam limiting material 7012 is round as common in image intensifier based c-arms. In this example of 7012 it is also demonstrated that this layer of beam limiting material is not limited to the standard geometries as conventional collimators and can assume any shape on the external rim as well as on the internal rim (circle in Fig. 7).

It would also be appreciated that this approach is applicable also to the iris that is a typical component and feature of c-arms equipped with an image intensifier detector. In such a case the beam limiting layer will typically assume a circular opening in accordance to the magnification level of the image intensifier or other dimension. It would be appreciated that also when replacing an iris, the opening is not limited to a circle and can take any shape.

It will also be appreciated that also exchangeable beam-spectrum modifying filters (such as beam hardening filters) that are typically included in the collimator assembly of many c-arms can be removed to provide space for the filters of the present invention and filters of the present invention can assume at least one layer of such a beam-spectrum modifying filter (such as aluminum or cupper or both).

When the dose reduction filter material is identical to the beam-spectrum modifying filter material, the two functions can be provided using a layer of the material where the ROI region is of a thickness as required for the beam-spectrum modifying filter and the filter outside of the ROI is thicker, as needed to reduce the dose outside the ROI. For example, the ROI region may be constructed from 0.5mm copper and the region outside the ROI may be constructed out of 1.3mm copper. A construction of such a filter can be made using a 1.3mm copper plate in which the ROI region is machined to reduce it's thickness to 0.5mm.

In yet another embodiment, two different materials can be used where a first material with a first thickness is used to construct the dose reduction filter with an ROI opening and a second material with a second thickness is cut to the shape of the ORI and is assembled in the ROI by welding or glue to provide the beam-spectrum modifying function. It would be appreciated that this embodiment can be realized with identical first and second materials (with different thicknesses) or with identical first and second thickness (with different materials).

It would be appreciated that a filter of the present invention can also be constructed from a layer of partially transparent filter with any external shape and with ROI opening of any shape, a layer of material that is relatively opaque to x-ray with any external shape and with ROI opening of any shape and at least one layer of beam-spectrum modifying filter.

Also, the combination of a layer of partially transparent filter with any external shape and with ROI opening of any shape and at least one layer of beam-spectrum modifying filter.

It would be appreciated that the above described embodiments and the invention are not limited to the case when parts have to be removed from the original collimator to provide for vacancy for the filters of the present invention and the filters of the present invention can also be implemented when removal of original collimator parts is not required.

## Claims

1. In a multiple frame x-ray imaging system, multiple stacked interchangeable filters mounted in a housing, each filter comprising a Region of Interest (ROI) aperture, said multiple filters comprising at least two filters having different Region of Interest (ROI) aperture shapes.

2. The system of claim 1, wherein said multiple filters include at least two filters providing different radiation dose reductions.

3. The system of claim 2, wherein said at least two filters providing different radiation dose reductions have different thicknesses.

4. The system of claim 2, wherein said at least two filters providing different radiation dose reductions are made of different materials.

5. The system of claim 1, wherein at least one of said multiple filters comprises a ROI with irregular shape.

6. The system of claim 1, further comprising a drawing mechanism configured to draw selected one or more filters in a first horizontal direction inside said housing for selectively reducing radiation.

7. The system of claim 6, wherein said drawing mechanism comprises independent slider crank mechanisms (1400, 1500, 1600) for each filter, said crank mechanisms (1400, 1500, 1600) driven by respective motors.

8. The system of claim 6, wherein said drawing mechanism comprises independent rack and pinion gear sets (2400, 2400A, 2500, 2500A) for each filter, said rack and pinion gear sets (2400, 2400A, 2500, 2500A), driven by respective motors (2700, 2800).

9. The system of claim 6, wherein said drawing mechanism comprises independent leadscrews for each filter, said leadscrews actuated by leadscrew actuators (3400, 3500, 3600).

10. The system of claim 6, wherein said selected one or more filters comprise more than one filter, whereby an active ROI aperture shape is created by the intersection of the ROI shapes of said more than one filter.

11. The system of claim 6, further comprising a mechanism for moving said housing in a second horizontal direction, essentially perpendicular to said first horizontal direction.

12. The system of claim 1, wherein at least one of said plurality of filters is replaceable.

13. The system of claim 6, wherein said drawing mechanism is configured to slide said filters so that the center of at least one ROI does not coincide with the center of the radiated beam.

14. The system of claim 1, wherein at least one of said filters comprises a hybrid of at least two materials.

15. The system of claim 14, wherein said at least two materials comprise a beam limiting material.
